# EUROPÄISCHE PATENTSCHRIFT

(11) **EP 1 654 243 B1**
(45) Veröffentlichungstag und Bekanntmachung des Hinweises auf die Patenterteilung: **05.09.2007**
(21) Anmeldenummer: 04740087.4
(22) Anmeldetag: 19.06.2004
(51) Int. Cl.: C07D 307/08

(54) **VERFAHREN ZUR GEWINNUNG VON OLIGOMEREN DES POLYTETRAHYDROFURANS ODER DER TETRAHYDROFURAN-COPOLYMERE**
METHOD FOR OBTAINING OLIGOMERS OF POLYTETRAHYDROFURANE OR TETRAHYDROFURANE COPOLYMERS
PROCEDE D'OBTENTION D'OLIGOMERES DE POLYTETRAHYDROFURANE OU DE COPOLYMERES DE TETRAHYDROFURANE

(30) Priorität: 08.07.2003 DE 10330721
(43) Veröffentlichungstag der Anmeldung: 10.05.2006
(73) Patentinhaber: BASF Aktiengesellschaft, 67056 Ludwigshafen (DE)
(72) Erfinder: MENGER, Volkmar, 67434 Neustadt (DE); SCHWARTZTRAUBER, Michael, 67435 Neustadt (DE); HAUBNER, Martin, 69124 Eppelheim (DE); PFAFF, Klaus-Peter, 67159 Friedelsheim (DE)
(86) Internationale Anmeldenummer: PCT/EP2004/006645
(87) Internationale Veröffentlichungsnummer: WO 2005/005405

(56) Entgegenhaltungen:
- DE-A- 4 410 685

## Beschreibung

Die Erfindung betrifft ein Verfahren zur Gewinnung von Oligomeren des Polytetrahydrofurans oder der Tetrahydrofuran-Copolymere aus einem bei der Umesterung der Mono- und/oder Diester des Polytetrahydrofurans oder der Tetrahydrofuran-Copolymere mit Methanol anfallenden methanolischen Rohprodukt durch zweistufige Destillation und anschließende Kondensation.

Polytetrahydrofuran - im folgenden PTHF genannt - das auch als Polyoxybutylenglykol bekannt ist, wird in der Kunststoff- und Kunstfaserindustrie als vielseitiges Zwischenprodukt verwendet und dient unter anderem zur Herstellung von Polyurethan-, Polyester- und Polyamid-Elastomeren. Daneben ist es, wie auch einige seiner Derivate, in vielen Anwendungsfeldern ein wertvoller Hilfsstoff, so zum Beispiel als Dispergiermittel oder beim Entfärben (Deinken) von Altpapier.

PTHF wird technisch üblicherweise durch Polymerisation von Tetrahydrofuran - im folgenden kurz THF genannt - an geeigneten Katalysatoren hergestellt. Durch Zugabe geeigneter Reagenzien kann die Kettenlänge der Polymerketten gesteuert werden und so das mittlere Molekulargewicht auf den gewünschten Wert eingestellt werden. Die Steuerung erfolgt dabei durch Wahl von Art und Menge des Telogens. Solche Reagenzien werden Kettenabbruchreagenzien oder "Telogene" genannt. Durch die Wahl geeigneter Telogene können zusätzlich funktionelle Gruppen an ein oder beiden Enden der Polymerkette eingeführt werden.

Andere Telegene wirken nicht nur als Kettenabbruchsreagenzien, sondern werden auch in die wachsende Polymerisatkette des PTHF's eingebaut. Sie haben nicht nur die Funktion eines Telogens, sondern sind gleichzeitig ein Comonomer und können daher mit gleicher Berechtigung sowohl als Telogene wie auch als Comonomere bezeichnet werden. Beispiele für solche Comonomere sind Telogene mit zwei Hydroxygruppen wie die Diole (Dialkohole). Dies können beispielsweise Ethylenglykol, Propylenglykol, Butylenglykol, 1,3-Propandiol, 2-Butin-1,4-diol, 1,6-Hexandiol oder niedermolekulares PTHF sein. Weiterhin sind als Comonomere cyclische Ether wie 1,2-Alkylenoxide, zum Beispiel Ethylenoxid oder Propylenoxid, 2-Methyltetrahydrofuran oder 3-Methyltetrahydrofuran geeignet. Die Verwendung solcher Comonomere führt mit Ausnahme von Wasser, 1,4-Butandiol und niedermolekularem PTHF zur Herstellung von Tetrahydrofuran-Copolymeren - im folgenden THF-Copolymere genannt - und ermöglicht es auf diese Weise, PTHF chemisch zu modifizieren.

Großtechnisch werden ganz überwiegend zweistufige Verfahren durchgeführt, bei denen Tetrahydrofuran z.B. in Gegenwart von Fluorsoulfonsäure zu Polytetrahydrofuran-Estern polymerisiert und anschließend zu Polytetrahydrofuran hydrolysiert wird.

Vorteilhaft ist insbesondere die THF-Polymerisation oder THF-Copolymerisation in Gegenwart von C₂- bis C₁₂-Carbonsäureanhydriden oder deren Gemischen mit C₂- bis C₁₂-Carbonsäuren wie z.B. Acetanhydrid oder Acetanhydrid-Essigsäure Gemische in Gegenwart saurer Katalysatoren zu Mono- und/oder Diestern des PTHF's oder der THF-Copolymere und anschließende basisch katalysierte Umesterung der PTHF-Ester oder Ester der THF-Copolymere mit Methanol zu PTHF oder THF-Copolymeren (mit endständigen Hydroxylgruppen), wie dies beispielsweise in EP-A 38009 und DE-A 2801578 beschrieben ist.

Das durch die Umesterung erhaltene methanolische Rohprodukt weist neben PTHF oder THF-Copolymeren eines mittleren Molekulargewichts von 500 bis 10.000 niedermolekulare Oligomere des PTHF oder der THF-Copolymere eines mittleren Molekulargewichts von 100 bis 500 auf. Es kann darüber hinaus weitere durch das Verfahren bedingte Verunreinigungen wie Natriumionen, die aus dem Umesterungskatalysator stammen, enthalten.

Die niedermolekularen Oligomere des PTHF oder der THF-Copolymere müssen abgetrennt werden, da sie sich auf die Polydispersität und die Farbzahl des PTHF's und der THF-Copolymere mit einem mittleren Molekulargewicht von 500 bis 10.000 auswirken.

Aufgabe der vorliegenden Erfindung ist es, ein Verfahren zur Gewinnung der niedermolekularen Polymere des PTHF's oder der THF-Copolymere zur Verfügung zu stellen, mit denen diese effektiv und mit möglichst geringem Aufwand aus dem nach der Umesterung erhaltenen methanolischen Rohprodukt abgetrennt werden können.

Zudem sollte das Verfahren es gestatten, die niedermolekularen Oligomere in einer Reinheit zu gewinnen, die ihre Depolymerisation zu THF bzw. THF und den entsprechenden Comonomeren und die anschließende Rückführung des durch die Rückspaltung erhaltenen THF's in die Polymerisation gestattet.

Es wurde nun ein Verfahren zur Gewinnung von Oligomeren des Polytetrahydrofurans oder der Tetrahydrofuran-Copolymere aus einem bei der Umesterung der Mono- und/oder Diester des Polytetrahydrofurans oder der Tetrahydrofuran-Copolymere mit Methanol anfallenden methanolischen Polytetrahydrofuran- oder Tetrahydrofuran-Copolymere enthaltenden Rohprodukt, gefunden, das dadurch gekennzeichnet ist, dass
a) aus dem Rohprodukt in einer ersten Destillationsstufe die Hauptmenge des Methanols abgetrennt und gegebenenfalls in die Umesterung zurückgeführt wird,
b) das erhaltene Sumpfprodukt destillativ in eine die Oligomere des Polytetrahydrofurans oder der Tetrahydrofuran-Copolymere enthaltende Kopffraktion und Polytetrahydrofuran oder Tetrahydrofuran-Copolymer aufgetrennt wird,
c) aus der Kopffraktion aus Stufe b) die Oligomere des Polytetrahydrofurans oder der Tetrahydrofuran-Copolymere auskondensiert werden.

Erfindungsgemäß wurde erkannt, dass die nach dem erfindungsgemäßen Verfahren gewonnenen Oligomere des PTHF's oder der THF-Copolymere einen Methanolgehalt unter 2 Gew.-%, bevorzugt ≤1,5 Gew.-%, aufweisen.

Aufgrund ihres niedrigen Methanolgehalts sind die erfindungsgemäß gewonnenen Oligomere für die aus dem Stand der Technik, beispielsweise aus der DE-A 4410685, bekannten Depolymerisationsverfahren, bei denen PTHF-Oligomere oder Oligomer des THF-Copolymers wieder in THF bzw. in THF und Comonomere zurückverwandelt werden, geeignet. Das durch die Depolymerisation erhaltene THF wird dann aus Gründen der Wirtschaftlichkeit in die Polymerisation des THFs zurückgefahren.

Da Methanol als ein den Kettenabbruch bewirkendes Telogen in der THF-Polymerisation wirkt und andererseits nicht in der Depolymerisation abgetrennt werden kann, ist es ein entscheidender Vorteil des erfindungsgemäßen Verfahrens, dass es Oligomere mit niedrigen Methanolgehalten liefert.

Als Edukt des erfindungsgemäßen Verfahrens dient ein PTHF oder THF-Copolymere enthaltendes methanolhaltiges Rohprodukt der Umesterung der Mono- und/oder Diester des PTHF's oder der THF-Copolymere, dessen Herstellung an sich aus dem Stand der Technik bekannt ist.

Bei diesem Herstellungsverfahren wird in einem ersten Schritt ein Mono- und/oder Diester des PTHF's bzw. der THF Copolymere durch Polymerisation von THF in Gegenwart von Telogenen und gegebenenfalls Comonomeren an sauren, bevorzugt heterogenen Katalysatoren, hergestellt.

Darunter seien beispielsweise genannt Katalysatoren auf Basis von Bleicherden wie sie beispielsweise in DE-A 1 226 560 beschrieben sind, genannt. Bleicherden, insbesondere auch aktivierte Montmorillonit, können als Formkörper im Festbett oder in Suspension verwendet werden.

Weiterhin sind Katalysatoren auf Basis gemischter Metalloxide für die Polymerisation von THF bekannt. So beschreibt die JP-A 04-306 228 die Polymerisation von THF in Gegenwart eines Carbonsäureanhydrids an einem gemischten Metalloxid bestehend aus Metalloxiden der Formel MₓO_{y} mit ganzzahligen x und y im Bereich 1-3. Genannt werden als Beispiele Al₂O₃-SiO₂, SiO₂-TiO₂, SiO₂-ZrO₂ und TiO₂-ZrO₂.

Die US 5,208,385 offenbart Katalysatoren auf Basis amorpher Silicium/Aluminium-Mischoxide. Auch Mischoxide auf Basis SnO₂/SiO₂, Ga₂O₃/SiO₂, Fe₂O₃/SiO₂, In₂O₃/SiO₂, Ta₂O₅/SiO₂ und HfO₂/SiO₂ sind bekannt. Die vorgenannten Katalysatoren werden bevorzugt durch Copräzipitation/Sol-Gel-Methoden hergestellt. Trägerkatalysatoren sind in der DE-A 44.33 606 offenbart, wobei Wolfram- oder Molybdänoxide auf z.B. ZrO₂, TiO₂, HfO₂, Y₂O₃, Fe₂O₃, Al₂O₃, SnO₂, SiO₂ oder ZnO aufgebracht werden. Weiterhin werden ZrO₂/SiO₂-Katalysatoren empfohlen, bei denen der Träger eine Alkalimetall-Konzentration < 5000 ppm aufweist.

Alle zitierten Katalysatoren können prinzipiell in Strangform als auch in Suspension eingesetzt werden.

Katalysatoren auf Basis saurer lonentauscher sind in US 4,120,903 für Polymerisation von THF, insbesondere alpha-Fluorsulfonsäure enthaltende Polymere (beispielsweise Nafion^{®}), in Gegenwart von Essigsäureanhydrid beschrieben. Weiterhin sind Katalysatoren, die ein Metall und Perfluoralkylsulfonsäure-Anionen enthalten für die THF-Polymerisation geeignet.

Daneben sind als Polymerisationskatalysatoren noch weitere gegebenenfalls aktivierte Tonmineralien bekannt, offenbart beispielsweise in der WO 94/05719, WO 96/23833, WO 98/51729, WO 99/12992 und DE-A 195 134 93. Auch Zeolithe sind als Katalysatoren geeignet und werden beispielsweise in DE-A 43 16 138 beschrieben. Schließlich sind noch sulfatisierte Zirkonoxide, sulfatisierte Aluminiumoxide, geträgerte Heteropolysäuren und geträgertes Ammoniumbifluorid (NH₄F*HF oder Antimonpentafluorid) als geeignete Polymerisationskatalysatoren bekannt. Bevorzugt wird das erfindungsgemäße Verfahren mit aktivierten Bleicherden durchgeführt.

Als Vorbehandlung des Katalysators kommen beispielsweise das Trocknen mit auf 80 bis 200°C, bevorzugt auf 100 bis 180°C erwärmten Gasen, wie z.B. Luft oder Stickstoff, in Frage.

Die Polymerisation wird im allgemeinen bei Temperaturen von 0 bis 80°C, vorzugsweise von 25°C bis zur Siedetemperatur des THF, durchgeführt. Der angewandte Druck ist in der Regel für das Ergebnis der Polymerisation nicht kritisch, weshalb im allgemeinen bei Atmosphärendruck oder unter dem Eigendruck des Polymerisationssystems gearbeitet wird.

Zur Vermeidung der Bildung von Etherperoxiden wird die Polymerisation vorteilhaft unter einer Inertgasatmosphäre vollzogen. Als Inertgase können z. B. Stickstoff, Kohlendioxid oder die Edelgase dienen, bevorzugt wird Stickstoff verwendet.

Das Verfahren kann diskontinuierlich oder kontinuierlich betrieben, wird jedoch aus wirtschaftlichen Gründen bevorzugt kontinuierlich betrieben.

Da das Telogen zum Kettenabbruch führt, lässt sich über die eingesetzte Telogenmenge das mittlere Molekulargewicht des herzustellenden Polymers steuern. Als Telogene eignen sich Carbonsäureanhydride und/oder Carbonsäuren bei der Herstellung von Mono- und Diestern des PolyTHF's. Vorzugsweise werden organische Carbonsäuren oder deren Anhydride verwendet. Unter diesen sind aliphatische und aromatische Poly- und/oder Monocarbonsäuren, die 2 bis 12, bevorzugt 2 bis 8 Kohlenstoffatome enthalten, bevorzugte Beispiele für aliphatische Carbonsäuren sind Essigsäure, Acrylsäure, Milchsäure, Propionsäure, Valeriansäure, Capronsäure, Caprylsäure und Pelargonsäure, von denen Essigsäure bevorzugt ist. Beispiele für aromatische Carbonsäuren sind Phthalsäure- und Naphthalincarbonsäure. Beispiele für Anhydride von aliphatischen Polycarbonsäuren sind Acrylsäure-Bernsteinsäure- und Maleinsäureanhydrid. Insbesondere ist Essigsäureanhydrid bevorzugt.

Die Konzentration des als Telogen eingesetzten Carbonsäureanhydrids in dem Polymerisationsreaktor zugeführten Eduktgemisch (Feed) liegt zwischen 0,03 bis 30 mol-%, bevorzugt bei 0,05 bis 20 mol-%, besonders bevorzugt bei 0,1 bis 10 mol-%, bezogen auf das eingesetzte THF. Wird zusätzlich eine Carbonsäure verwendet, so beträgt das Molverhältnis im Feed der laufenden Polymerisation üblicherweise 1 : 20 bis 1 : 20000, bezogen auf eingesetztes Carbonsäureanhydrid.

Die Mono- und Diester der THF-Copolymere lassen sich herstellen durch die zusätzliche Verwendung von cyclischen Ethern als Comonomere, die sich ringöffnend polymerisieren lassen, bevorzugt drei-, vier- und fünfgliedrige Ringe, wie 1,2-Alkylenoxide, z.B. Ethylenoxid oder Propylenoxid, Oxetan, substituierte Oxetane, wie 3,3-Dimethyloxetan, die THF-Derivate 2-Methyltetrahydrofuran oder 3-Methyltetrahydrofuran, wobei 2-Methyltetrahydrofuran oder 3-Methyltetrahydrofuran besonders bevorzugt sind.

Ebenso ist die Verwendung von C₂- bis C₁₂-Diolen als Comonomeren möglich. Dies können beispielsweise Ethylenglykol, Propylenglykol, Butylenglykol, 1,3-Propandiol, 2-Butin-1,4-diol, 1,6-Hexandiol oder niedermolekulares PTHF sein. Weiterhin sind als Comonomere cyclische Ether wie 1,2-Alkylenoxide, zum Beispiel Ethylenoxid oder Propylenoxid, 2-Methyltetrahydrofuran oder 3-Methyltetrahydrofuran geeignet.

Je nach Telogengehalt der Polymerisationsmischung lassen sich mit dem Verfahren Mono- und/oder Diester des PTHF oder der THF-Copolymere mit mittleren Molekulargewichten von 250 bis 10000 Dalton gezielt herstellen, vorzugsweise werden mit dem erfindungsgemäßen Verfahren die betreffenden PTHF-Ester mit mittleren Molekulargewichten von 500 bis 5000 Dalton, besonders bevorzugt 650 bis 4000 Dalton hergestellt. Unter der Bezeichnung "mittleres Molekulargewicht" oder "mittlere Molmasse" wird in dieser Anmeldung das Zahlenmittel Mₙ des Molekulargewichts der Polymere verstanden, dessen Bestimmung durch nasschemische OH-Zahlbestimmung erfolgt.

Der THF-haltige Austrag aus der Polymerisationsstufe wird filtriert um Spuren des Polymerisationskatalysators zurückzuhalten und im Anschluss der destillativen THF-Abtrennung zugeführt. Es kann jedoch auch zuerst THF abgetrennt werden und dann die verbleibenden Mono- oder Diester des PTHFs von Katalysatorrückständen durch Filtration befreit werden. Die zweite Methode gilt als bevorzugt. Als Filtereinrichtungen werden industriell übliche Schichtenfilter verwendet.

Die Estergruppen in den so erhaltenen Polymeren müssen in einem zweiten Schritt umgewandelt werden. Eine übliche hier verwendete Methode stellt die durch alkalische Katalysatoren initiierte Umsetzung mit niederen Alkoholen dar. Die Umesterung mit alkalischen Katalysatoren ist aus dem Stand der Technik bekannt und beispielsweise in DE-A 101 20 801 und DE-A 197 42 342 beschrieben.

Bei der Herstellung des methanolischen Rohproduktes für das erfindungsgemäße Verfahren wird Methanol als niederer Alkohol verwendet. Als wirksamer Umesterungskatalysator wird Natriummethylat verwendet.

Dementsprechend werden die durch die Polymerisation erhaltenen Mono- oder Diester des PTHF oder der THF-Copolymere für die Umesterung zunächst mit Methanol versetzt. Der Gehalt an Mono- und/oder Diacetat im Methanol sollte zwischen 20 und 80 Gew.-% liegen. Dann wird Natriummethylat in einer Menge von 50 bis 1000 ppm zugegeben.

Da das nach der Umesterung erhaltene methanolische Rohprodukt noch Natriumionen aus dem Umesterungskatalysator enthalten kann, ist es bevorzugt, das Rohprodukt in Gegenwart einer katalytischen Menge Wasser zunächst direkt durch zumindestens einen lonentauscher zu leiten. Die Durchführung dieser lonentauscherbehandlung ist in DE-A 197 58 296, auf die hier ausdrücklich verwiesen wird, offenbart. Bevorzugt wird ein gelförmiger, starksaurer lonentauscher verwendet. Das vom Katalysator befreite methanolische Rohprodukt wird bevorzugt noch über einen industriell üblichen Simplexfilter filtriert und dann dem erfindungsgemäßen Verfahren zugeführt.

Die erste Destillation des methanolischen Rohprodukts (Stufe a)) wird bei schwachem Überdruck, bevorzugt bei 20 mbar bis 500 mbar Überdruck, besonders bevorzugt bei 20 bis 250 mbar und insbesondere bevorzugt bei 20 bis 150 mbar Überdruck betrieben. Die Temperatur liegt bei dieser Destillation zwischen 50 und 250°C, bevorzugt zwischen 50 und 200°C und besonders bevorzugt bei 100 bis 190°C. In dieser ersten Destillationsstufe wird die Hauptmenge des im Rohprodukt enthaltenen Methanols, mehr als 95 %, abgetrennt. Die Destillation wird in an sich bekannten Destillationsapparaten bevorzugt in einer Verdampferstufe durchgeführt. Das Methanol wird über Kopf abgetrennt, während als Sumpfprodukt eine PTHF oder THF-Copolymere eines Molekulargewichts von 500 bis 10000 und die niedermolekularen Oligomere des PTHF's und der THF-Copolymere enthaltende Fraktion erhalten wird. Das abgetrennte Methanol wird bevorzugt in die Umesterung zurückgeführt.

Das in Stufe a) erhaltene Sumpfprodukt hingegen, die nach einem Restgehalt an Methanol von bevorzugt weniger als 5 Gew.-% aufweist, wird der zweiten Destillationsstufe zugeführt. Die zweite Destillation (Stufe b)) wird bei einem absoluten Druck von 1 mbar bis 300 mbar, bevorzugt 1 mbar bis 100 mbar und besonders bevorzugt bei 1 mbar bis 20 mbar bei einer Temperatur von 50 bis 250°C, bevorzugt 50 bis 200°C und besonders bevorzugt zwischen 100 und 190°C betrieben. Die Destillation der Stufe b) wird in an sich bekannten Destillationsapparaten, bevorzugt in einer Verdampferstufe durchgeführt. In Stufe b) werden die niedermolekularen Oligomere des PTHF's oder der THF-Copolymere zusammen mit geringen Spuren von Methanol über Kopf abgetrennt. Als Sumpfprodukt werden PTHF oder THF-Copolymere mit einem mittleren Molekulargewicht von 500 bis 10000 abgetrennt.

Aus der Kopffraktion der Stufe b) werden dann bei 5 bis 40°C, bevorzugt an einem mit entsprechend temperierten Kühlwasser gekühlten Kondensator, die niedermolekularen Oligomere des PTHF's oder THF-Copolymere auskondensiert. Aufgrund ihres geringen Methanolgehalts von weniger als 2 Gew.-%, bevorzugt ≤1,5 Gew.-%, werden sie bevorzugt in die Depolymerisation verbracht. Das durch die Depolymerisation zurückgewonnene THF kann dann erneut zur Polymerisation verwendet werden.

Aus dem nach der Kondensation der niedermolekularen Oligomere des PTHF's oder der THF-Copolymere verbleibenden Produkt kann durch erneute Kondensation bei -40°C, beispielsweise mit einem mit Sole gekühlten Kondensator, das restliche Methanol durch Kondensation zurückgewonnen werden und in die Umesterung rückgeführt werden.

Die Erfindung wird im weiteren anhand von Beispielen näher erläutert.

### Beispiele

### Molekulargewichtsbestimmung

### Bestimmung der OH-Zahl

Unter der Hydroxyzahl wird diejenige Menge an Kaliumhydroxid in mg verstanden, die der bei der Acetylierung von 1 g Substanz gebundenen Menge Essigsäure äquivalent ist.

Die Hydroxyzahl wird bestimmt durch die Veresterung der vorhandenen Hydroxylgruppe mit einem Überschuss an Essigsäureanhydrid. Nach der Umsetzung wird das überschüssige Essigsäureanhydrid gemäß folgender Reaktionsgleichung mit Wasser hydrolysiert und als Essigsäure mit Natronlauge zurücktitriert.

### Bestimmung Eindampfrückstand

### Methode 1 (EDR 1)

Zur Bestimmung des Eindampfrückstands wurde die zuvor gewogene Probe am Rotationsverdampfer 1 h lang bei 140°C und bei Normaldruck und dann 1 h lang bei gleicher Temperatur bei 0,1 bis 0,2 mbar behandelt und erneut gewogen. Der Eindampfrückstand ergab sich als das prozentuale Gewichtsverhältnis zum Anfangswert.

### Methode 2 (EDR 2)

Zur Bestimmung des Eindampfrückstandes wurde die zuvor gewogene Probe am Rotationsverdampfer 1 h lang bei 140°C und Normaldruck und sodann 1 h bei 140°C und 125 mbar behandelt. Der Eindampfrückstand ergab sich als das prozentuale Gewichtsverhältnis zum Anfangswert.

### Beispiel 1

5000 kg/h einer 60 gew.-%igen Lösung von PTHF eines mittleren Molekulargewichts von 800 wurden in eine bei einem Überdruck von 100 mbar und 170°C betriebene erste Destillationsstufe gefahren und dort in einem Brüdenstrom von 2000 kg/h Methanol und einem Sumpfstrom von 3000 kg/h (Gew.-%: > 95 Roh-PTHF, < 5 Methanol, bestimmt nach EDR 2) aufgetrennt.

Der Sumpfaustrag der ersten Destillation wurde in eine zweite bei einem Absolutdruck von 10 mbar und 170°C betriebene Destillationsstufe gefahren und dort in einen den Restgehalt an Methanol und niedrigsiedende Oligomere des PTHF's aufweisenden Kopfstrom und den Sumpfstrom aus PTHF eines mittleren Molekulargewichts von ca. 800 getrennt.

Der Brüdenstrom der zweiten Destillation wurde mit auf 25°C temperiertem Kühlwasser an einen Kondensator kondensiert. Die erhaltenen 10 kg/h des Kondensats, das 98,5 Gew.-% Oligomere des PTHF's (Mₙ ca. 200) und 1,5 Gew.-% Methanol (bestimmt nach EDR 1) aufwies, konnte der Depolymerisation zugeführt werden. Durch eine erneute Kondensation bei -40°C des verbleibenden Brüdenstroms werden Reste des Methanols auskondensiert und in die Umesterung zurückgeführt.

### Beispiel 2

10000 kg/h einer 60 gew.-%igen Lösung von PTHF eines mittleren Molekulargewichts von 1600 wurden bei einem Überdruck von 100 mbar und 170°C betriebene erste Destillationsstufe gefahren und dort in einem Brüdenstrom von 4000 kg/h Methanol und einem Sumpfstrom von 6000 kg/h (Gew.-%: > 95 Roh-PTHF, < 5 Methanol (bestimmt nach EDR 2) aufgetrennt.

Der Sumpfaustrag der ersten Destillation wurde in eine zweite bei einem Absolutdruck von 10 mbar und 170°C betriebene Destillationsstufe gefahren und dort in einen den Restgehalt an Methanol und niedrigsiedende Oligomere des PTHF's aufweisenden Kopfstrom und den Sumpfstrom aus PTHF eines mittleren Molekulargewichts von ca. 1600 getrennt.

Der Kopfstrom der zweiten Destillation wurde mit auf 25°C temperiertem Kühlwasser an einem Kondensator kondensiert. Die erhaltenen 5 kg/h des Kondensats, das 98,5 Gew.-% Oligomere des PTHF's (Mₙ ca. 200) und 1,5 Gew.-% Methanol (bestimmt nach EDR 1) aufwies, konnte direkt der Depolymerisation zugeführt werden. Durch eine erneute Kondensation des verbleibenden Brüdenstroms bei -40°C wurden Reste des Methanols auskondensiert und in die Umesterung zurückgeführt.

## Patentansprüche

1. Verfahren zur Gewinnung von Oligomeren des Polytetrahydrofurans oder der Tetrahydrofuran-Copolymere aus einem bei der Umesterung der Mono- und/oder Diester des Polytetrahydrofurans oder der Tetrahydrofuran-Copolymere mit Methanol anfallenden methanolischen Polytetrahydrofuran- oder Tetrahydrofuran-Copolymere enthaltenden Rohprodukt, **dadurch gekennzeichnet, dass**
a) aus dem Rohprodukt in einer ersten Destillationsstufe die Hauptmenge des Methanols abgetrennt,
b) das erhaltene Sumpfprodukt destillativ in eine die Oligomere des Polytetrahydrofurans oder der Tetrahydrofuran-Copolymere enthaltende Kopffraktion und Polytetrahydrofuran oder Tetrahydrofuran-Copolymer aufgetrennt wird,
c) aus der Kopffraktion aus Stufe b) die Oligomere des Polytetrahydrofurans oder der Tetrahydrofuran-Copolymere auskondensiert werden.

2. Verfahren nach Anspruch 1, **dadurch gekennzeichnet, dass** das in Stufe a) abgetrennte Methanol in die Umesterung zurückgeführt wird.

3. Verfahren nach einem der Ansprüche 1 oder 2, **dadurch gekennzeichnet, dass** in der Stufe a) bei 20 bis 500 mbar Überdruck und einer Temperatur von 50 bis 250°C destilliert wird.

4. Verfahren nach einem der Ansprüche 1 bis 3, **dadurch gekennzeichnet, dass** in Stufe b) bei einem Absolutdruck von 1 mbar bis 300 mbar und bei 50 bis 250°C destilliert wird.

5. Verfahren nach einem der Ansprüche 1 bis 4, **dadurch gekennzeichnet, dass** bei einer Temperatur von 5 bis 40°C in Stufe c) kondensiert wird.

6. Verfahren nach einem der Ansprüche 1 bis 5, **dadurch gekennzeichnet, dass** das erhaltene Rohprodukt vor Stufe a) von aus dem Umesterungskatalysator stammenden Natriumionen durch eine Behandlung mit einem lonentauscher befreit wird.

## Claims

1. A process for obtaining oligomers of polytetrahydrofuran or of tetrahydrofuran copolymers from a methanolic crude product which comprises polytetrahydrofuran or tetrahydrofuran copolymers and is obtained in the transesterification of the mono- and/or diesters of polytetrahydrofuran or tetrahydrofuran copolymers with methanol, which comprises
a) removing the majority of the methanol from the crude product in a first distillation stage,
b) separating the resulting bottom product by distillation into a top fraction comprising the oligomers of polytetrahydrofuran or of tetrahydrofuran copolymers, and polytetrahydrofuran or tetrahydrofuran copolymer,
c) condensing the oligomers of polytetrahydrofuran or of tetrahydrofuran copolymers out of the top fraction from stage b).

2. The process according to claim 1, wherein the methanol removed in stage a) is recycled into the transesterification.

3. The process according to either of claims 1 and 2, wherein distillation is effected in stage a) at from 20 to 500 mbar gauge and a temperature of from 50 to 250°C.

4. The process according to any of claims 1 to 3, wherein distillation is effected in stage b) at an absolute pressure of from 1 to 300 mbar and at from 50 to 250°C.

5. The process according to any of claims 1 to 4, wherein condensation is effected in stage c) at a temperature of from 5 to 40°C.

6. The process according to any of claims 1 to 5, wherein the crude product obtained is freed before stage a) of sodium ions stemming from the transesterification catalyst by treatment with an ion exchanger.

## Revendications

1. Procédé d'obtention d'oligomères du polytétrahydrofuranne ou des copolymères du tétrahydrofuranne à partir d'un produit brut contenant des copolymères méthanoliques du polytétrahydrofuranne ou du tétrahydrofuranne qui apparaissent lors de la transestérification des monoesters et/ou des diesters du polytétrahydrofuranne ou des copolymères du tétrahydrofuranne avec du méthanol, **caractérisé en ce que** :
a) la quantité principale du méthanol est séparée du produit brut dans une première étape de distillation,
b) le produit de pied obtenu est séparé par distillation en une fraction de tête contenant les oligomères du polytétrahydrofuranne ou des copolymères du tétrahydrofuranne et en du polytétrahydrofuranne ou des copolymères du tétrahydrofuranne,
c) les oligomères du polytétrahydrofuranne ou des copolymères de tétrahydrofuranne sont extraits par condensation à partir de la fraction de tête de l'étape b).

2. Procédé selon la revendication 1, **caractérisé en ce que** le méthanol séparé dans l'étape a) est recyclé dans la transestérification.

3. Procédé selon l'une des revendications 1 ou 2, **caractérisé en ce que** la distillation s'effectue dans l'étape a) à une surpression de 20 mbars à 500 mbars et à une température de 50 à 250°C.

4. Procédé selon l'une quelconque des revendications 1 à 3, **caractérisé en ce que** la distillation s'effectue dans l'étape b) à une pression absolue de 1 mbar à 300 mbars et entre 50 et 250°C.

5. Procédé selon l'une quelconque des revendications 1 à 4, **caractérisé en ce que** la condensation s'effectue dans l'étape c) à une température de 5 à 40°C.

6. Procédé selon l'une quelconque des revendications 1 à 5, **caractérisé en ce que** le produit brut obtenu est débarrassé avant l'étape a) des ions sodium provenant du catalyseur de transestérification grâce à un traitement avec un échangeur d'ions.
